# EUROPÄISCHE PATENTANMELDUNG

(11) **EP 4 091 573 A1**
(43) Veröffentlichungstag der Anmeldung: **23.11.2022**
(21) Anmeldenummer: 22169703.0
(22) Anmeldetag: 25.04.2022
(51) Int. Cl.: A61B 90/30, A61B 90/00, A61B 17/00, A61B 34/20

(54) **OPERATIONSLEUCHTENANORDNUNG MIT AUTOMATISCH AUSRICHTBARER KAMERA**

(30) Priorität: 04.05.2021 DE 102021111459
(71) Anmelder: Karl Leibinger Medizintechnik GmbH & Co. KG, 78570 Mühlheim (DE)
(72) Erfinder: STRÖLIN, Joachim, 78604 Rietheim-Weilheim (DE)
(74) Vertreter: Behr, Wolfgang

(57) **Zusammenfassung**

Die vorliegende Erfindung betrifft eine Operationsleuchtenanordnung (1), welche mindestens eine Operationsleuchte (2) zum Beleuchten eines Operationsfeldes (F) umfasst, mit mindestens einer Kamera (10) zum Aufnehmen eines Bildes (11) des Operationsfeldes (F). Die Operationsleuchtenanordnung (1) weist eine Steuerung (20) auf, welche das Bild (11) der Kamera (10) automatisch ausrichtet.

## Beschreibung

Die vorliegende Erfindung betrifft eine Operationsleuchtenanordnung, welche mindestens eine Operationsleuchte umfasst, mit mindestens einer Kamera.

Ein Operationsraum ist häufig mit einer auf das Operationsfeld ausgerichteten Kamera ausgerüstet, wobei das Bild der Kamera auf einen Monitor im Operationsraum oder in einem externen Raum für beispielsweise Schulungszwecke übertragen wird. Dadurch kann das Operationsfeld auch für Personen sichtbar gemacht werden, die keinen direkten Blick in das Operationsfeld haben. Ebenso ist es denkbar, dass das Bild der Kamera dem Operateur bei der Durchführung der Operation dient. Zudem kann das Bild der Kamera zu Dokumentationszwecken aufgezeichnet werden.

Es ist üblich, die Kamera an einer Operationsleuchte vorzusehen, wobei die Operationsleuchte dazu dient, das Operationsfeld auszuleuchten. Beim Ausrichten der Operationsleuchte wird jedoch zunächst keine Rücksicht auf die Ausrichtung der Kamera genommen. Dies kann zu der unerwünschten Situation führen, dass das Bild der Kamera nicht der gewünschten Ausrichtung entspricht, beispielsweise einer Blickrichtung des Operateurs, und das Operationsfeld verdreht darstellt ist. Das Bild kann beispielsweise auf dem Kopf stehen. Dies macht eine Orientierung auf dem Bild der Kamera nahezu unmöglich.

Zur Lösung dieses Problems wurde oder wird die Kamera bisher manuell ausgerichtet. Nachteilig ist hierbei, dass neben dem manuellen Ausrichten der Operationsleuchte ein zusätzliches manuelles Ausrichten der Kamera nötig ist.

Aufgabe der vorliegenden Erfindung ist es daher, eine verbesserte Operationsleuchtenanordnung bereitzustellen.

Diese Aufgabe wird durch eine Operationsleuchtenanordnung gemäß den Ansprüchen 1 bis 13 und/oder durch ein Set aus einer Operationsleuchtenanordnung und einem Symbol gemäß Anspruch 14 und/oder einem Verfahren zum Betrieb einer Operationsleuchtenanordnung gemäß Anspruch 15 gelöst. Bevorzugte Ausgestaltungen der vorliegenden Erfindung ergeben sich aus den jeweiligen Unteransprüchen.

Die vorliegende Erfindung umfasst in einem ersten Aspekt eine Operationsleuchtenanordnung, welche mindestens eine Operationsleuchte zum Beleuchten eines Operationsfeldes umfasst, mit mindestens einer Kamera zum Aufnehmen eines Bildes des Operationsfeldes. Eine Steuerung der Operationsleuchtenanordnung ist so ausgestaltet, dass ein Bild der Kamera automatisch ausgerichtet wird.

Durch ein automatisches Ausrichten des Bildes kann der bisher zum manuellen Ausrichten notwendige Aufwand vermieden werden. Weiterhin kann beispielsweise vermieden werden, dass durch eine nicht-korrekt ausgerichtete Kamera der Betrachter des Bildes sich auf dem Bild nicht orientieren kann. Weiterhin wird die beim manuellen Ausrichten bestehende Problematik umgangen, dass entweder eine Steuerung im sterilen Bereich vorgesehen sein muss oder der Operateur die Kamera nicht selbst ausrichten kann.

In einer möglichen Ausgestaltung der vorliegenden Erfindung ist die Kamera an der Operationsleuchte, insbesondere an einem Griff der Operationsleuchte, angeordnet.

In einer möglichen Ausgestaltung der vorliegenden Erfindung ist die Kamera an einem Tragesystem der Operationsleuchtenanordnung angeordnet.

In einer möglichen Ausgestaltung der vorliegenden Erfindung ist die Ausrichtung der optischen Hauptachse der Kamera gegenüber der optischen Hauptachse der Operationsleuchte nicht automatisch verstellbar. Gegebenenfalls kann eine Ausrichtung der optischen Hauptachse der Kamera gegenüber der optischen Hauptachse der Operationsleuchte jedoch manuell verstellbar sein.

Bevorzugt kommt die vorliegende Erfindung jedoch in Operationsleuchtenanordnungen zum Einsatz, bei welchen die Ausrichtung der optischen Hauptachse der Kamera gegenüber der optischen Hauptachse der Operationsleuchte konstruktiv fest vorgegeben ist.

In einer möglichen Ausgestaltung der vorliegenden Erfindung verläuft eine optische Hauptachse der Kamera parallel oder in einem festen Winkel zu einer optischen Hauptachse der Operationsleuchte. Insbesondere ist die relative Ausrichtung von Operationsleuchte und Kamera so gewählt, dass die Kamera ein Bild des von der Operationsleuchte beleuchteten Bereichs aufnimmt.

In einer möglichen Ausgestaltung der vorliegenden Erfindung fällt die optische Hauptachse der Kamera mit der optischen Hauptachse der Operationsleuchte zusammen.

Insbesondere kann die Kamera an einem mittig an der Operationsleuchte angeordneten Griff angeordnet sein und in die gleiche Richtung ausgerichtet sein wie die Operationsleuchte.

In einer möglichen Ausgestaltung der vorliegenden Erfindung erfolgt das Ausrichten des Bildes der Kamera durch Ausrichten der Kamera.

In einer möglichen Ausgestaltung der vorliegenden Erfindung erfolgt das Ausrichten des Bildes der Kamera durch digitales Ausrichten des Bildes.

Insbesondere kann das Ausrichten des Bildes der Kamera mittels Drehen der Kamera um mindestens eine Achse erfolgen.

In einer möglichen Ausgestaltung der vorliegenden Erfindung erfolgt das Ausrichten des Bildes der Kamera mittels Drehen der Kamera um ihre optische Hauptachse.

In einer möglichen Ausgestaltung der vorliegenden Erfindung erfolgt das Ausrichten des Bildes der Kamera mittels digitalem Drehen des Bildes, insbesondere durch digitales Drehen des Bildes in der Bildebene bzw. um eine Achse, welche senkrecht auf der Bildebene steht.

In einer möglichen Ausgestaltung der vorliegenden Erfindung umfasst die Operationsleuchtenanordnung einen Antrieb, der mittels der Steuerung so angesteuert werden kann, dass er ein Ausrichten der Kamera, insbesondere ein Drehen der Kamera um ihre optische Hauptachse, bewirkt, sodass das Bild der Kamera ausgerichtet wird.

In einer möglichen Ausgestaltung der vorliegenden Erfindung ist der Antrieb an der Operationsleuchte angeordnet.

In einer möglichen Ausgestaltung der vorliegenden Erfindung ist der Antrieb in die Operationsleuchte integriert ist.

Insbesondere kann der Antrieb im Griff der Operationsleuchte angeordnet sein und die Kamera um ihre optische Hauptachse, welche mit der optischen Hauptachse der Operationsleuchte zusammenfällt, drehen.

In einer möglichen Ausgestaltung der vorliegenden Erfindung ermittelt die Steuerung eine Ausrichtung der Kamera mittels eines Neigungssensors.

In einer möglichen Ausgestaltung der vorliegenden Erfindung ist der Neigungssensor an der Operationsleuchte vorgesehen.

In einer möglichen Ausgestaltung der vorliegenden Erfindung ist der Neigungssensor an einem Tragesystem der Operationsleuchtenanordnung vorgesehen.

Der Neigungssensor ermittelt die Neigung gegenüber der durch die Schwerkraft vorgegebenen Lotrichtung und daher eine absolute Ausrichtung des Elementes, an welchem er angeordnet ist im Raum bestimmt, insbesondere die absolute Ausrichtung der Operationsleuchte.

In einer möglichen Ausgestaltung der vorliegenden Erfindung ermittelt die Steuerung eine Ausrichtung der Kamera mittels einer Objekterfassung zum Erfassen von Objekten in dem Bild der Kamera.

In einer möglichen Ausgestaltung der vorliegenden Erfindung ermittelt die Steuerung in mindestens einem Betriebsmodus anhand von Signalen eines Neigungssensors eine Soll-Ausrichtung und richtet das Bild entsprechend automatisch aus.

In einer möglichen Ausgestaltung der vorliegenden Erfindung ist in der Soll-Ausrichtung das Bild der Kamera so ausgerichtet, dass eine Oberkante des Bildes der Kamera parallel zur Horizontalen ausgerichtet ist.

In einer möglichen Ausgestaltung der vorliegenden Erfindung ermittelt die Steuerung in mindestens einem Betriebsmodus mittels einer Objekterfassung eine Soll-Ausrichtung und richtet das Bild entsprechend automatisch aus.

In einer möglichen Ausgestaltung der vorliegenden Erfindung erfasst die Objekterfassung ein oder mehrere Objekte im Bild der Kamera.

Insbesondere kann die Objekterfassung eine Bilderkennungssoftware umfassen. Insbesondere kann es sich bei der Objekterfassung um eine Bilderkennungssoftware handeln.

In einer möglichen Ausgestaltung der vorliegenden Erfindung handelt es sich bei dem Objekt um ein Symbol, insbesondere einen Pfeil, das eine Richtung definiert, wobei die Steuerung das Bild entsprechend dieser Richtung automatisch ausrichtet.

In einer möglichen Ausgestaltung der vorliegenden Erfindung entspricht, wenn sich das Bild der Kamera in der Soll-Ausrichtung befindet, die Richtung des Symbols einer Richtung von einer Unterkante zu einer Oberkante des Bildes.

In einer möglichen Ausgestaltung der vorliegenden Erfindung entspricht, wenn sich das Bild der Kamera in der Soll-Ausrichtung befindet, die Richtung des Symbols der Vertikalen des Bildes.

In einer möglichen Ausgestaltung der vorliegenden Erfindung ist das Symbol neben einem Operationsfeld auf einem Patienten abgelegt und/oder an einem medizinischen Instrument angeordnet.

In einer weiteren möglichen Ausgestaltung der vorliegenden Erfindung handelt es sich bei dem Objekt um eine Person, insbesondere einen Operateur.

In einer möglichen Ausgestaltung der vorliegenden Erfindung richtet die Steuerung das Bild entsprechend einer Blickrichtung der Person und/oder einer Geste der Person automatisch aus.

In einer weiteren möglichen Ausgestaltung der vorliegenden Erfindung handelt es sich bei dem Objekt um ein medizinisches Instrument und/oder um eine Anordnung aus mehreren medizinischen Instrumenten, beispielsweise einen oder mehrere Wundhaken, wobei die Steuerung das Bild entsprechend der Ausrichtung des Instruments und/oder der Anordnung aus mehreren medizinischen Instrumenten ausrichtet.

In einer weiteren möglichen Ausgestaltung der vorliegenden Erfindung wird durch die Objekterfassung eine OP-Situation erkannt, wobei die Steuerung das Bild entsprechend der erkannten OP-Situation ausrichtet.

Beispielsweise werden die beteiligten Personen und deren Anordnung um den Patienten erkannt und das Bild gemäß einer Soll-Ausrichtung für die erkannte OP-Situation ausgerichtet. In der Steuerung können mehrere OP-Situationen zusammen mit einer jeweiligen Soll-Ausrichtung des Bildes abgespeichert sein. In einer möglichen Ausgestaltung der vorliegenden Erfindung ist in mindestens einem Betriebsmodus das Bild manuell ausrichtbar und/oder richtet die Steuerung in mindestens einem Betriebsmodus anhand von Signalen einer manuell bedienbaren Eingabevorrichtung das Bild aus.

Weiterhin kann in mindestens einem Betriebsmodus das Bild manuell ausrichtbar sein, wobei das manuelle Ausrichten unabhängig von der Steuerung erfolgt, insbesondere durch manuelles Drehen der Kamera.

In einer möglichen Ausgestaltung der vorliegenden Erfindung umfasst die Operationsleuchtenanordnung mindestens zwei Betriebsmodi, wobei in einem ersten Betriebsmodus die Ausrichtung des Bildes automatisch erfolgt und in einem zweiten Betriebsmodus die Ausrichtung des Bildes manuell erfolgt. Im ersten Betriebsmodus kann die Ausrichtung anhand von Signalen eines Neigungssensors erfolgen und/oder mittels einer Objekterfassung.

In einer möglichen Ausgestaltung ist die Steuerung so ausgeführt, dass ein Bediener zwischen mehreren unterschiedlichen Betriebsmodi zum automatischen und/oder manuellen Ausrichten des Bildes auswählen kann.

Insbesondere kann die Operationsleuchtenanordnung so ausgestaltet sein, dass mittels manueller Bedienung einer Eingabevorrichtung zwischen mindestens zwei Betriebsmodi ausgewählt werden kann.

In einem zweiten Aspekt umfasst die vorliegende Erfindung ein Set aus einer Operationsleuchtenanordnung und einem Symbol, welches von einer Objekterfassung der Steuerung erfasst und zum Ausrichten des Bildes herangezogen wird, wobei das Symbol bevorzugt ein sterilisierbares Element darstellt.

Insbesondere kann es sich bei dem Symbol um ein Einwegprodukt handeln.

In einem dritten unabhängigen Aspekt umfasst die vorliegende Erfindung ein Verfahren zum Betrieb einer Operationsleuchtenanordnung, wobei die Operationsleuchtenanordnung mindestens eine Operationsleuchte und mindestens eine Kamera umfasst. Das Verfahren umfasst folgende Schritte:
- Beleuchten eines Operationsfeldes mittels der Operationsleuchte;
- Aufnehmen eines Bildes des Operationsfeldes mittels der Kamera; und
- automatisches Ausrichten des Bildes der Kamera, insbesondere durch ein Drehen des Bildes der Kamera, insbesondere durch automatisches Drehen um eine Hauptachse der Kamera.

In einer möglichen Ausgestaltung der vorliegenden Erfindung erfolgt die automatische Ausrichtung über einen Neigungssensor und/oder eine Objekterfassung zum automatischen Ausrichten, insbesondere Drehen, des Bildes der Kamera mittels der Steuerung.

Das Verfahren erfolgt bevorzugt so, wie dies oben im Hinblick auf die erfindungsgemäße Operationsleuchtenanordnung bereits beschrieben wurde.

Die vorliegende Erfindung wird nun anhand von Figuren sowie Ausführungsbeispielen näher erläutert.

Dabei zeigen:
- Fig. 1: ein Ausführungsbeispiel einer erfindungsgemäßen Operationsleuchtenanordnung mit einer Kamera;
- Fig. 2: das in Fig. 1 gezeigte Ausführungsbeispiel einer erfindungsgemäßen Operationsleuchtenanordnung, wobei ein Bild, welches mit einer der Kameras aufgenommen wurde, mit verdrehter Ausrichtung angezeigt wird;
- Fig. 3: das Ausführungsbeispiel aus Fig. 2, wobei das Bild entsprechend einer Soll-Ausrichtung ausgerichtet wurde.

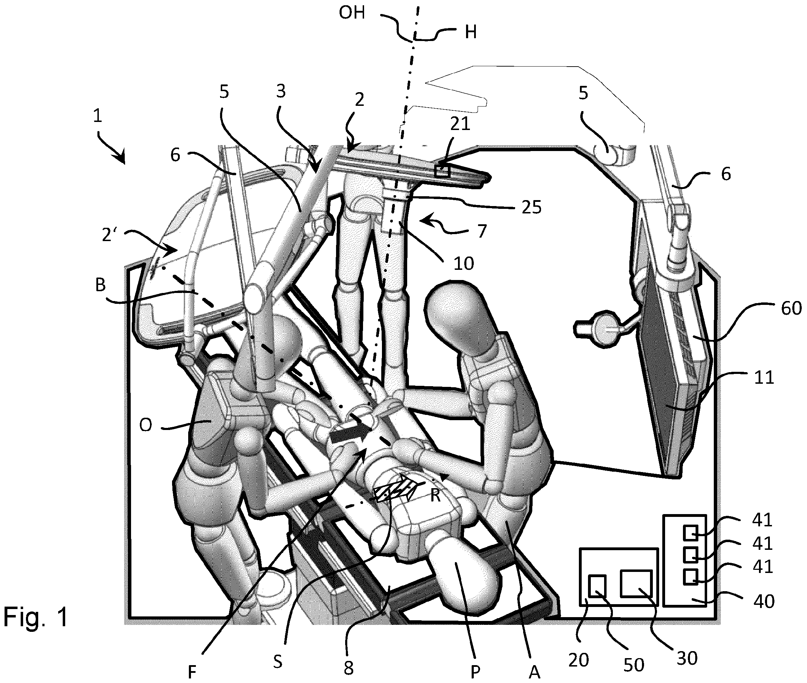

Fig. 1 bis Fig. 3 zeigen jeweils ein Ausführungsbeispiel einer erfindungsgemäßen Operationsleuchtenanordnung 1 mit einer ersten Operationsleuchte 2 und einer zweiten Operationsleuchte 2'. Die Operationsleuchtenanordnung 1 könnte im Rahmen der vorliegenden Erfindung jedoch auch nur eine Operationsleuchte 2 oder mehr als zwei Operationsleuchten 2 umfassen.

Im jeweiligen Ausführungsbeispiel ist ein Tragesystem 3 vorgesehen, an welchem die Operationsleuchten 2 und 2' oberhalb eines Operationstisches 8 angeordnet sind. Die Operationsleuchten 2 und 2' können in ihrer Position und Ausrichtung verstellt werden. Ein Verstellen kann manuell erfolgen. Des Weiteren kann eine Verstellung durch einen oder mehrere Antriebe des Tragesystems 3 realisiert werden.

Im jeweiligen Ausführungsbeispiel umfasst das Tragesystem 3 eine Deckenhalterung (nicht dargestellt), an der eine Zentralwelle (nicht dargestellt) angeordnet sein kann. An der Zentralwelle wiederum können Tragarme 5 verschwenkbar angeordnet sein, wobei die Tragarme bevorzugt um eine durch die Zentralwelle gebildete vertikal verlaufende Achse verschwenkbar sind. Die Operationsleuchten 2 und 2' können jeweils über weitere Tragarmelemente 6 und/oder Gelenke an unterschiedlichen Tragarmen 5 angeordnet sein. Andere Ausgestaltungen des Tragesystems 3 als die soeben beschriebene sind ebenso denkbar.

Die in Fig. 1 bis Fig. 3 dargestellten Operationsleuchten 2 und 2' erzeugen jeweils ein Lichtfeld. Die Operationsleuchten 2 und 2' können nun jeweils so ausgerichtet und/oder positioniert werden, dass ein Operationsfeld F eines Patienten P, der auf dem Operationstisch 8 liegt, wie gewünscht beleuchtet wird. In anderen Fällen können die Lichtfelder der beiden Operationsleuchten 2 und 2' auch auf unterschiedliche Bereiche ausgerichtet sein. Dies ist beispielsweise bei einer Transplantation denkbar, wobei eine Operationsleuchte 2 auf das Operationsfeld F gerichtet sein kann, während eine Operationsleuchte 2' auf das zu transplantierende Organ gerichtet sein kann.

In dem in Fig. 1 bis 3 dargestellten Ausführungsbeispiel weisen die Operationsleuchten 2 und 2' einen Griff 7 und 7' zur Positionierung und Ausrichtung der jeweiligen Operationsleuchte 2 und 2' auf. In Fig. 1 ist nur der Griff 7 der Operationsleuchte 2 ersichtlich. Im Ausführungsbeispiel von Fig. 1 ist der Griff 7 mittig an der Operationsleuchte 2 angeordnet. Der Griff 7 ist insbesondere mittig an einem Leuchtenkörper angeordnet und von Licht aussendenden Bereichen der Leuchte umgeben. Die Hauptachse des Griffs 7 bzw. 7' entspricht bevorzugt der optischen Hauptachse der Leuchte 2 bzw. 2'.

Der Griff 7 bzw. 7' kann weiterhin, wie in den in Fig. 1 bis 3 dargestellten Ausführungsbeispielen gezeigt, im geometrischen Zentrum einer das Lichtfeld erzeugenden Lichtquelle 2a' der entsprechenden Operationsleuchte 2 bzw. 2' angeordnet sein. Wie in Fig. 1 bis 3 dargestellt, kann die Operationsleuchte 2 bzw. 2' auch einen oder mehrere Griffelemente, die seitlich an der Operationsleuchte 2 bzw. 2' angeordnet sind, aufweisen. Die Griffelemente können alternativ oder zusätzlich zum Griff 7 bzw. 7' vorgesehen sein.

In dem in Fig. 1 bis 3 dargestellten Ausführungsbeispiel umfasst die Operationsleuchtenanordnung 1 eine Kamera 10 zur Erzeugung eines Bildes 11, wobei die Operationsleuchtenanordnung 1 weiterhin einen Monitor 60 zur Anzeige des Bildes 11 der Kamera 10 aufweist. Die Kamera 10 ist an der ersten Operationsleuchte 2 angeordnet.

In einer ersten Variante weist die Operationsleuchte 2' keine Kamera auf.
in einer zweiten Variante umfasst die Operationsleuchtenanordnung 1 dagegen zwei Kameras 10 und 10', die entsprechend an den Operationsleuchten 2 und 2' angeordnet sind.

Der Monitor 60 kann wie auch die Operationsleuchten 2 und 2' über ein Tragarmelement 6 am Tragesystem 3 angeordnet sein. In den in Fig. 1 bis 3 dargestellten Ausführungsbeispielen befindet sich der Monitor 60 im Operationsraum. Es ist allerdings auch denkbar, dass der Monitor 60 oder ein anderer Monitor in einem vom Operationsraum separaten Raum vorgesehen ist. Somit kann das Bild 11 der Kamera 10 beispielsweise zu Schulungszwecken zur Verfügung gestellt werden. Es ist auch denkbar, dass Bild der Kamera zu Dokumentationszwecken einer Speichereinheit zuzuführen und aufzuzeichnen.

Der Begriff "Bild" im Sinne der vorliegenden Erfindung umfasst sowohl ein Videobild im Sinne einer Bildsequenz als auch ein einzelnes Bild im Sinne eines Fotos.

In einer möglichen Ausgestaltung der vorliegenden Erfindung handelt es sich bei dem Bild um ein Videobild.

Wie in Fig. 1 bis 3 dargestellt sind im Ausführungsbeispiel die optische Hauptachse OH der Kamera 10 und die optische Hauptachse H der Operationsleuchte 2 parallel zueinander ausgerichtet und fallen darüber hinaus zusammen. Wie in Fig. 1 bis 3 dargestellt kann die optische Hauptachse H der Operationsleuchte 2 durch ein geometrisches Zentrum der Operationsleuchte 2, insbesondere durch ein geometrisches Zentrum einer Lichtquelle der Operationsleuchte 2 verlaufen, insbesondere durch einen Mittelpunkt der Operationsleuchte 2.

Die Operationsleuchte 2, insbesondere deren Lichtquelle, kann betrachtet entlang ihrer Hauptachse eine rechteckige und/oder runde Form aufweisen und/oder ist tellerförmig ausgebildet.

Wie in Fig. 1 bis 3 dargestellt entspricht die optische Hauptachse H der Operationsleuchte 2 einer Mittelachse eines durch die Lichtquelle erzeugten Lichtkegels. Weisen beide Operationsleuchten 2, 2' jeweils eine Kamera 10, 10' auf, fällt die optische Hauptachse der jeweiligen Kamera 10, 10' bevorzugt mit der optischen Hauptachse der jeweiligen Operationsleuchte 2' zusammen.

Gemäß der vorliegenden Erfindung umfasst die Operationsleuchtenanordnung eine Steuerung, die so ausgestaltet ist, dass ein Bild der Kamera automatisch ausgerichtet wird.

In Fig. 1 bis 3 weist die jeweilige Operationsleuchtenanordnung 1 eine Steuerung 20 auf, mittels derer das Bild 11 der Kamera 10, 10' automatisch ausgerichtet wird. Dies kann mittels Ausrichten der Kamera 10 bzw. 10' und/oder mittels digitalem Ausrichten des Bildes 11 bzw. 11' der Kamera 10 bzw. 10' erfolgen. Die digitale Ausrichtung des Bildes 11 bzw. 11' kann mittels der Steuerung 20 erfolgen, insbesondere mittels einer Recheneinheit 50, die orzugsweise in die Steuerung 20 integriert ist. Die jeweilige Operationsleuchte 2 kann kabelgebunden und/oder kabellos mit der Steuerung 20 und/oder der Eingabevorrichtung 40 vernetzt sein.

Bevorzugt erfolgt das Ausrichten des Bildes 11 mittels Ausrichten der Kamera 10, 10', d.h. durch eine mechanische Ausrichtung der Kamera 10, 10', wobei die mechanische Ausrichtung bevorzugt mittels des beispielsweise in Fig. 1 dargestellten Antriebs 25, 25' erfolgt. In dem in Fig. 1 bis 3 dargestellten Ausführungsbeispiel ist der Antrieb 25 an der Operationsleuchte 2 angeordnet. Insbesondere kann der Antrieb 25 wie in Fig. 1 dargestellt in den Griff 7 der Operationsleuchte 2 integriert sein. Mittels der Steuerung 20 kann der Antrieb 25, 25' so angesteuert werden, dass die Kamera 10, 10' entsprechend einer Soll-Ausrichtung des Bildes 11, 11' ausgerichtet wird.

Im Ausführungsbeispiel kann die Kamera 10, 10' um ihre optische Hauptachse OH gedreht werden, um das Bild 11 auszurichten. Die Steuerung 20 steuert hierfür den Antrieb 25, 25' so an, dass durch die Ausrichtung der Kamera 10, 10' eine Soll-Ausrichtung des Bildes 11, 11' der Kamera 10, 10' erzielt wird.

Alternativ oder zusätzlich kann die automatische Ausrichtung des Bildes 11 bzw. 11' mittels digitalem Ausrichten des Bildes 11 bzw. 11' erfolgen, insbesondere durch Drehen des Bildes in der Bildebene.

Die automatische Ausrichtung des Bildes kann auf unterschiedliche Art und Weise erfolgen:
In einer ersten Ausgestaltung ermittelt die Steuerung 20 anhand von Signalen eines Neigungssensors 21 die Ausrichtung der Kamera 10, 10'. Der Neigungssensor 21 kann wie beispielsweise in Fig. 1 bis 3 dargestellt an der Operationsleuchte 2, 2' angeordnet sein. Es ist aber auch denkbar, den Neigungssensor 21 an einer anderen Stelle vorzusehen, beispielsweise am Tragarm 5. Bei dem Neigungssensor 21 handelt es sich bevorzugt um eine digitale und/oder elektronische Wasserwaage.

Bevorzugt erfolgt die Ausrichtung des Bildes 11 bzw. 11' anhand des Neigungssensors 21 bzw. 21' so, dass eine Oberkante des Bildes 11 bzw. 11' parallel zu einer Horizontalen ausgerichtet ist.

In einer zweiten Ausgestaltung ist eine Objekterfassung 30 vorgesehen, mittels welcher die Steuerung 20 die Ausrichtung des Bildes 11, 11' der Kamera 10, 10'ermitteln kann, wie beispielsweise in Fig. 1 bis 3 gezeigt. Die automatische Ausrichtung des Bildes 11 bzw. 11' erfolgt dann in eine anhand der Objekterfassung 30 ermittelte Soll-Ausrichtung.

Bei der Objekterfassung 30 kann es sich beispielsweise um eine Bilderkennungssoftware handeln. Die Steuerung 20 kann die Objekterfassung 30 umfassen, insbesondere kann die Objekterfassung 30 integraler Bestandteil der Steuerung 20 sein. Die Objekterfassung 30 kann aber auch separat zur Steuerung 20 vorgehen sein. Die Objekterfassung 30 kann derart ausgebildet sein, dass sie ein oder mehrere Objekte im Bild 11 der Kamera 10 bzw. im Bild 11' der Kamera 10' erfassen kann.

Die Objekterfassung 30 kann eine oder mehrere der folgenden Funktionen aufweisen:
a. Über das aufgenommene Bild 11 bzw. 11' der Kamera 10 bzw. 10' wird die OP-Situation erkannt und das Bild 11 bzw. 11' danach ausgerichtet. Beispielsweise werden die beteiligten Personen O bzw. A und deren Anordnung um den Patienten P erkannt und das Bild 11 bzw. 11' gemäß einer Soll-Ausrichtung für die erkannte OP-Situation ausgerichtet. In der Steuerung 20 können mehrere OP-Situationen zusammen mit einer jeweiligen Soll-Ausrichtung des Bildes 11 bzw. 11' abgespeichert sein.
b. Über das aufgenommene Bild 11 bzw. 11' wird der Operateur O erkannt und das Bild 11 bzw. 11' entsprechend seiner Blickrichtung B ausgerichtet. Insbesondere kann erkannt werden, auf welcher Seite des Operationsfeldes F sich der Operateur O befindet, und das Bild 11 bzw. 11' so ausgerichtet werden, dass die Vertikale des Bildes 11 bzw. 11' einer Verbindungslinie zwischen der Position des Operateurs O und dem Operationsfeld F entspricht. Die Richtung der Verbindungslinie zwischen der Position des Operateurs O und dem Operationsfeld F kann dabei als Blickrichtung des Operateurs O definiert werden. Alternativ kann die tatsächliche Blickrichtung des Operateurs erkannt und der automatischen Ausrichtung zugrunde gelegt werden.
c. Über das aufgenommene Bild 11 bzw. 11' wird ein medizinisches Instrument (z.B. Wundhaken, Klemme oder Endoskop) erkannt und entsprechend dessen Richtung und/oder Lage das Bild 11 bzw. 11' ausgerichtet.
d. Über das aufgenommene Bild 11 bzw. 11' wird ein Hilfsmittel / Tool erkannt, nach dem das Bild 11 bzw. 11' ausgerichtet werden soll. Dies kann z. B. ein Richtungszeiger sein, welcher bevorzugt sterilisierbar oder als Einmalprodukt ausgeführt ist. Oder es kann die Ausrichtung eines Passiv-Panels, welches zur Ansteuerung der Leuchtenanordnung 1 und/oder anderer Geräte im Operationssaal ohnehin erfasst wird, zur Ausrichtung des Bildes 11 bzw. 11' der Kamera 10 bzw. 10' herangezogen werden.

Bei dem Passiv-Panel gemäß Variante d) kann es sich insbesondere um ein passives Bedienpanel handeln, wie es aus der DE 10 2020 114 426 der Anmelderin bekannt ist. Das Bedienpanel kann ein passives, steriles und/oder sterilisierbares Element sein. Auf dem Bedienpanel kann mindestens ein Symbol vorgesehen sein, mittels welchem Bedienereingaben auf dem Bedienpanel vorgenommen werden. Es kann sich bei dem Bedienpanel um ein komplett passives Bedienpanel handeln, d.h. es besitzt keine elektronischen Komponenten oder eine Stromversorgung. Bevorzugt ist es hergestellt aus einem sterilisierbaren Kunststoff oder Edelstahl. Aus diesen Eigenschaften heraus ist es ohne Probleme sterilisierbar und wird wie die normalen Instrumente wie Skalpelle, Klemmen, Scheren usw. sterilisiert aufbereitet. Denkbar ist aufgrund des sehr einfachen Aufbaus auch eine Einweg-Variante.

Bei Punkt a.-d. erfolgt die Auswertung über eine Bilderkennung. Daraus resultierend wird der Antrieb 25 bzw. 25' der Kamera 10 bzw. 10' gesteuert und das Bild 11 bzw. 11' automatisch ausgerichtet. Es ist auch möglich das Bild 11 bzw. 11' digital auszurichten/drehen, hierzu ist kein Antrieb 25 bzw. 25' notwendig. Die Drehung wird dann ausschließlich digital realisiert.

Wie in Fig. 1 dargestellt kann gemäß Variante d) neben dem Operationsfeld F ein Symbol S, bei dem es sich wie dargestellt um einen Pfeil handeln kann, vorgesehen sein. Im Ausführungsbeispiel erfasst die Objekterfassung 30 im Bild 11 der Kamera 10 das Symbol S und ermittelt anhand der Ausrichtung R des Symbols S die Ausrichtung der Kamera 10 bzw. die Ausrichtung des Bildes 11. Die Steuerung 20 richtet sodann anhand der ermittelten Ausrichtung R des Symbols S das Bild 11 der Kamera 10 entsprechend einer Soll-Ausrichtung aus. Das Symbol S kann beispielsweise auch an einem medizinischen Instrument vorgesehen sein. Beispielsweise kann das Bild 11 so ausgerichtet werden, dass das Symbol S in eine definierte Richtung zeigt, beispielsweise nach oben.

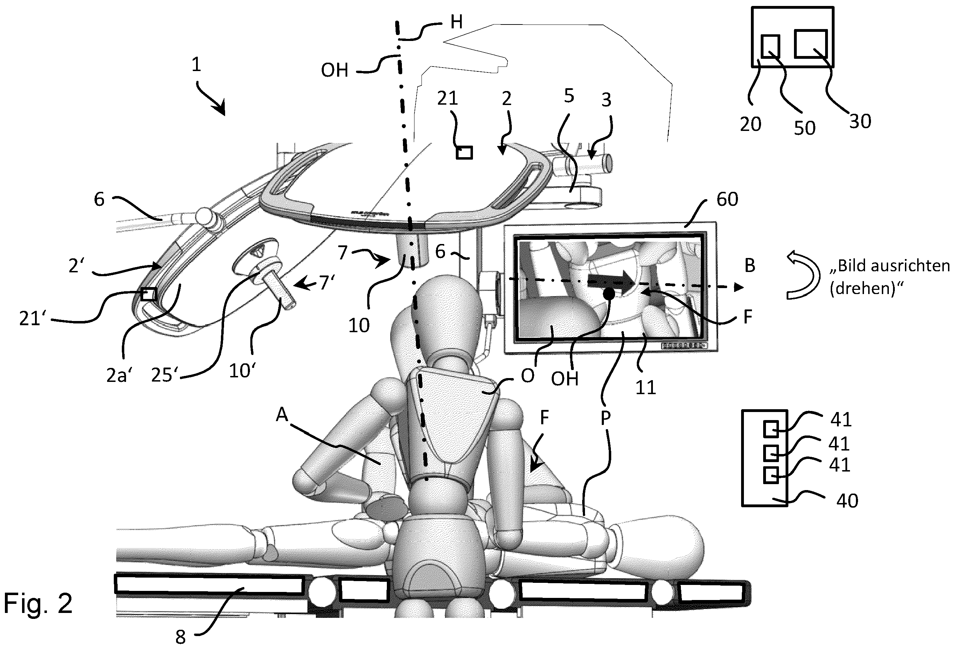

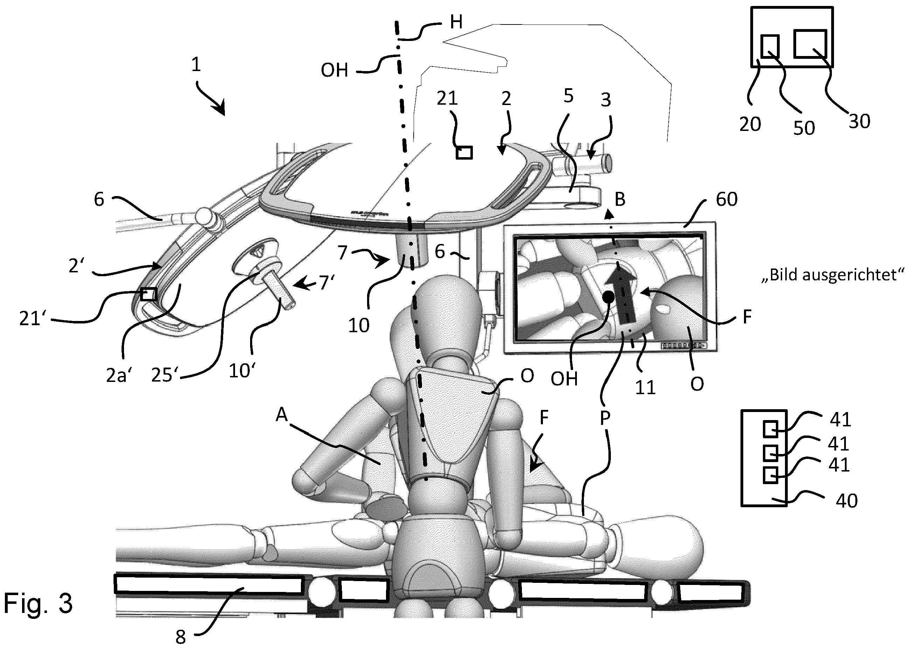

Fig. 2 zeigt ein Bild 11 der Kamera 10 in einer Ausrichtung, die nicht seiner Soll-Ausrichtung entspricht. Fig. 3 hingegen zeigt ein Bild 11 der Kamera 10, das entsprechend einer Soll-Ausrichtung ausgerichtet ist. Das in Fig. 3 dargestellte Bild 11 wurde durch ein erfindungsgemäßes Ausrichten, insbesondere Drehen des in Fig. 2 dargestellten Bildes 11 erhalten. Das Drehen des Bildes 11 kann durch Drehen der Kamera 10 oder durch digitales Drehen des Bildes der Kamera erfolgen.

In einer zweiten Variante erfasst die Objekterfassung 30 den Operateur O auf dem Bild 11 der Kamera 10. Insbesondere kann die Objekterfassung 30 die Blickrichtung B des Operateurs O erfassen, wobei das Bild 11 der Kamera 10 entsprechend der Blickrichtung B mittels der Steuerung 20 ausgerichtet wird. Es ist auch denkbar, dass das Bild 10 der Kamera 11 entsprechend einer erfassten Geste des Operateurs O ausgerichtet wird.

Die in Fig. 2 und Fig. 3 dargestellten Bilder 11 und 11 zeigen jeweils ein Operationsfeld F, wobei mittels eines Pfeils die Blickrichtung B des Operateurs O veranschaulicht ist. Des Weiteren ist ein Patient P abgebildet.

Die optische Hauptachse OH der Kamera, über welche das Bild 11 aufgenommen wurde, verläuft in beiden Figuren senkrecht zur Bildebene der Anzeige und damit in den Figuren zur Zeichenebene. Wie in Fig. 2 und 3 gezeigt, kann die optische Hauptachse OH der Kamera, um welche das Bild gedreht wird, durch das geometrische Zentrum, insbesondere den Mittelpunkt des Bildes 11 verlaufen.

Ein Ausrichten/Drehen des Bildes 11 kann beispielsweise durch Drehen der Kamera 10 und/oder mittels digitalem Drehen des Bildes 11 der Kamera 10 erfolgen.

In dem in Fig. 3 dargestellten Ausführungsbeispiel entspricht die Soll-Ausrichtung einer Ausrichtung des Bildes 11, in der die Blickrichtung B des mittels der Bilderfassung 30 erfassten Operateurs O von einer Unterkante zu einer Oberkante des Bildes 11 verläuft.

Die Operationsleuchtenanordnung kann mehrere Betriebsmodi umfassen, in welchen die automatische Ausrichtung jeweils anhand unterschiedlicher Kriterien erfolgt. Das jeweils einzusetzende Kriterium kann bevorzugt durch einen Bediender der Operationsleuchtenanordnung auswählbar sein.

Das in Fig. 1 bis 3 gezeigte Ausführungsbeispiel der Operationsleuchtenanordnung 1 umfasst einen ersten Betriebsmodus, in dem das Ausrichten des Bildes 11, 11' automatisch anhand von Signalen des Neigungssensors 21 erfolgt. Im Ausführungsbeispiel umfasst die Operationsleuchtenanordnung 1 weiterhin einen zweiten Betriebsmodus, in dem das Ausrichten des Bildes 11 automatisch mittels der Objekterfassung 30 erfolgt. Im Ausführungsbeispiel umfasst die Operationsleuchtenanordnung 1 einen dritten Betriebsmodus, in dem das Ausrichten des Bildes 11 manuell erfolgt.

In einem Ausführungsbeispiel der Operationsleuchtenanordnung 1 umfassend zwei Kameras 10 und 10' kann das Ausrichten des jeweiligen Bildes 11, 11' unabhängig voneinander entsprechend unterschiedlicher Betriebsmodi oder entsprechend einem gemeinsamen Betriebsmodus erfolgen.

Im ersten Betriebsmodus der in Fig. 1 bis 3 dargestellten Operationsleuchtenanordnung 1 ermittelt deren Steuerung 20 die Ausrichtung der Kamera 10 mittels des Neigungssensors 21, der an der Operationsleuchte 2 angeordnet ist. Anhand der Signale des Neigungssensors 21 ermittelt die Steuerung 20 eine Soll-Ausrichtung des Bildes 11 und richtet das Bild 11 entsprechend automatisch aus.

Im zweiten Betriebsmodus der in Fig. 1 bis 3 dargestellten Operationsleuchtenanordnung 1 wird die Ausrichtung des Bildes 11 mittels der Objekterfassung 30 ermittelt. Mittels der Objekterfassung 30 wird eine Soll-Ausrichtung des Bildes 11 ermittelt, wobei das Bild 11 entsprechend automatisch ausgerichtet wird.

Der zweite Betriebsmodus kann mehrere Untermodi umfassen, welche sich im Hinblick auf die Kriterien, nach welchen aufgrund der Objekterfassung 30 eine automatische Ausrichtung erfolgt, unterscheiden. Insbesondere kann je nach Untermodus die automatische Ausrichtung anhand unterschiedlicher von der Objekterfassung ausgewerteter Kriterien erfolgen, insbesondere anhand der Kriterien a) bis d) oben.

Sowohl im ersten als auch im zweiten Betriebsmodus kann das automatische Ausrichten des Bildes 11 mittels der Steuerung 20 durch Ansteuerung des Antriebes 25 und einem damit korrespondierenden mechanischen Ausrichten der Kamera 10 erfolgen.

Im Ausführungsbeispiel wird hierzu die Kamera 10 um ihre optische Hauptachse OH gedreht, sodass die Ausrichtung der Kamera 10 der jeweiligen Soll-Ausrichtung des Bildes 11 der Kamera 10 entspricht.

Alternativ oder zusätzlich kann das automatische Ausrichten des Bildes 11 mittels digitalem Ausrichten, insbesondere Drehen des Bildes 11 erfolgen. Das digitale Ausrichten des Bildes 11 kann mittels der Steuerung 20 erfolgen, insbesondere mittels einer Recheneinheit 50 der Steuerung 20. Alternativ kann das digitale Ausrichten des Bildes 11 auch mittels einer Recheneinheit 50 erfolgen, die separat von der Steuerung 20 vorgesehen sein kann. Die Steuerung 20 kann mit der Recheneinheit 50 kabellos und/oder kabelgebunden kommunizieren.

Im dritten Betriebsmodus der in Fig. 1 bis 3 dargestellten Operationsleuchtenanordnung 1 erfolgt die Ausrichtung des Bildes 11 manuell. Im Ausführungsbeispiel erfolgt dies mittels manueller Betätigung der Eingabevorrichtung 40.

Im Ausführungsbeispiel kommuniziert die Eingabevorrichtung 40 mit der Steuerung 20, wobei die Steuerung 20 das Bild 11 anhand von Signalen der Eingabevorrichtung 40 ausrichtet. Das manuelle Ausrichten des Bildes 11 kann mittels Ausrichten der Kamera 10, insbesondere Drehen der Kamera 10 um ihre optische Hauptachse OH erfolgen und/oder mittels digitalem Ausrichten des Bildes 11, insbesondere mittels Drehen der Bildes 11 um seine optische Hauptachse OH.

Es ist auch denkbar, die Operationsleuchtenanordnung 1 so auszugestalten, dass in einem Betriebsmodus ein manuelles Ausrichten des Bildes 11 in Kombination mit einem automatischen Ausrichten des Bildes 11 erfolgt. In einem solchen Betriebsmodus könnte die Steuerung 20 die beiden Arten des Ausrichtens aufeinander abstimmen. Es wäre beispielsweise denkbar, dass ein manuelles Ausrichten des Bildes 11 nur zur Nachjustierung herangezogen wird, beispielsweise nachdem ein automatisches Ausrichten des Bildes 11 erfolgt ist.

Bevorzugt kann ein Bediener zwischen den unterschiedlichen Betriebsmodi der Operationsleuchtenanordnung auswählen.

Bevorzugt kann ein Bediener zwischen unterschiedlichen Betriebsmodi der Operationsleuchtenanordnung derart auswählen, dass mindestens zwei Bilder unterschiedlicher Kameras entsprechend unterschiedlichen Betriebsmodi oder entsprechend einem gemeinsamen Betriebsmodus ausgerichtet werden.

Zum Wechseln zwischen den verschiedenen Betriebsmodi kann die in Fig. 1 bis 3 dargestellte Eingabevorrichtung 40 vorgesehen sein, mittels deren manueller Bedienung die verschiedenen Betriebsmodi ausgewählt werden können.

Die in Fig. 1 bis 3 dargestellte Eingabevorrichtung 40 kann hierzu mindestens ein Eingabeelement 41 aufweisen. In den in Fig. 1 bis 3 gezeigten Ausführungsbeispielen ist die Eingabevorrichtung 40 weiterhin derart ausgestaltet, dass die manuelle Ausrichtung des Bildes 11 über die manuelle Betätigung der Eingabevorrichtung 40 erfolgt. Hierfür können an der Eingabevorrichtung 40 entsprechende Eingabeelemente 41 vorgesehen sein, die beispielsweise in Form von Schaltern, Reglern und/oder eines Touchscreens vorliegen können. Beispielsweise kann ein erstes Eingabeelement 41 zum Drehen des Bildes 11 links um dessen optische Hauptachse und ein zweites Eingabeelement zum Drehen des Bildes 11 rechts um dessen optische Hauptachse vorgesehen sein.

Sind zwei Kameras 10 und 10' vorgesehen, kann das jeweilige Ausrichten der Bilder 11, 11' wie bereits zuvor beschrieben erfolgen.

In einer bevorzugten Ausgestaltung der vorliegenden Erfindung werden die Bilder 11, 11' der beiden Kameras 10 und 10' automatisch so ausgerichtet, dass sie das Operationsfeld F in der gleichen Ausrichtung zeigen.

Weiterhin kann die Operationsleuchtenanordnung 1 derart ausgebildet sein, dass mittels der Eingabevorrichtung 40 manuell ausgewählt werden kann, welches Bild 11, 11' oder welche Bilder auf dem Monitor 60 angezeigt werden. Beispielsweise kann somit mittels manueller Betätigung der Eingabevorrichtung 40 mindestens ein Bild aus den Bildern 11 und 11' zur Anzeige auf dem Monitor 60 ausgewählt werden. Die Bilder können auf dem Monitor 60 beispielsweise auch nebeneinander angeordnet werden.

### Bezugszeichenliste:

- 1: Operationsleuchtenanordnung
- 2, 2': Operationsleuchte
- 2a': Lichtquelle der Operationsleuchte 2'
- 3: Tragesystem
- 5: Tragarm
- 6: Tragarmelement
- 7: Griff
- 8: Operationstisch

- 10, 10': Kamera
- 11, 11': Bild der Kamera

- 20: Steuerung
- 21: Neigungssensor
- 25: Antrieb

- 30: Objekterfassung
- 40: Eingabevorrichtung
- 41: Eingabeelemente
- 50: Recheneinheit
- 60: Monitor

- F: Operationsfeld
- OH: Optische Hauptachse der Kamera 10
- H: Optische Hauptachse der Operationsleuchte 2
- S: Symbol
- R: Richtung
- O: Operateur
- B: Blickrichtung
- A: Assistenz
- P: Patient

## Patentansprüche

1. Operationsleuchtenanordnung (1), welche mindestens eine Operationsleuchte (2) zum Beleuchten eines Operationsfeldes (F) umfasst, mit mindestens einer Kamera (10) zum Aufnehmen eines Bildes (11) des Operationsfeldes (F), **dadurch gekennzeichnet,**
**dass** die Operationsleuchtenanordnung (1) eine Steuerung (20) aufweist, welche das Bild (11) der Kamera (10) automatisch ausrichtet.

2. Operationsleuchtenanordnung (1) nach Anspruch 1, wobei die Kamera (10) an der Operationsleuchte (2), insbesondere an einem Griff (7) der Operationsleuchte (2) angeordnet ist, oder wobei die Kamera (10) an einem Tragesystem (3) der Operationsleuchtenanordnung (1) angeordnet ist.

3. Operationsleuchtenanordnung (1) nach einem der vorangegangenen Ansprüche, wobei eine optische Hauptachse (OH) der Kamera (10) parallel oder in einem festen Winkel zu einer optischen Hauptachse (H) der Operationsleuchte (2) verläuft, wobei die optische Hauptachse (OH) der Kamera (10) bevorzugt mit einer optischen Hauptachse (H) der Operationsleuchte (2) zusammenfällt, und/oder wobei eine Ausrichtung der optischen Hauptachse (OH) der Kamera (10) gegenüber der optischen Hauptachse (H) der Operationsleuchte (2) konstruktiv fest vorgegeben und/oder nicht automatisch verstellbar ist.

4. Operationsleuchtenanordnung (1) nach einem der vorangegangenen Ansprüche, wobei ein Ausrichten des Bildes (11) der Kamera (10) mittels Ausrichten der Kamera (10) erfolgt und/oder mittels digitalem Ausrichten des Bildes (11) erfolgt, wobei vorzugsweise das Ausrichten des Bildes (11) der Kamera (10) mittels Drehen der Kamera (10) um ihre optische Hauptachse (OH) erfolgt und/oder mittels digitalem Drehen des Bildes (11) in der Bildebene erfolgt.

5. Operationsleuchtenanordnung (1) nach einem der vorangegangenen Ansprüche, weiterhin umfassend einen Antrieb (25), der mittels der Steuerung (20) so angesteuert werden kann, dass er ein Ausrichten der Kamera (10), insbesondere ein Drehen der Kamera (10) um ihre optische Hauptachse (OH), bewirkt, sodass das Bild (11) der Kamera (10) ausgerichtet wird, wobei der Antrieb (25) bevorzugt an der Operationsleuchte (2) angeordnet, insbesondere in diese integriert ist.

6. Operationsleuchtenanordnung (1) nach einem der vorangegangenen Ansprüche, wobei die Steuerung (20) eine Ausrichtung der Kamera (10) mittels eines Neigungssensors (21) ermittelt, der vorzugsweise an der Operationsleuchte (2) und/oder an einem Tragesystem (3) der Operationsleuchtenanordnung (1) vorgesehen ist, und/oder wobei die Steuerung (20) eine Ausrichtung der Kamera (10) mittels einer Objekterfassung (30) zum Erfassen von Objekten (S, O) in dem Bild (11) der Kamera (10) ermittelt.

7. Operationsleuchtenanordnung (1) nach einem der vorangegangenen Ansprüche, wobei die Steuerung (20) in mindestens einem Betriebsmodus anhand von Signalen eines Neigungssensors (21) eine Soll-Ausrichtung ermittelt und das Bild (11) entsprechend automatisch ausrichtet.

8. Operationsleuchtenanordnung (1) nach Anspruch 7, wobei in der Soll-Ausrichtung das Bild (11) der Kamera (10) so ausgerichtet ist, dass eine Oberkante des Bildes (11) der Kamera (10) parallel zur Horizontalen ausgerichtet ist.

9. Operationsleuchtenanordnung (1) nach einem der vorangegangenen Ansprüche, wobei die Steuerung (20) in mindestens einem Betriebsmodus mittels einer Objekterfassung (30) eine Soll-Ausrichtung ermittelt und das Bild (11) entsprechend automatisch ausrichtet, wobei die Objekterfassung (30), insbesondere eine Bilderkennungssoftware, bevorzugt ein oder mehrere Objekte (S, O) im Bild (11) der Kamera (10) erfasst.

10. Operationsleuchtenanordnung (1) nach Anspruch 9, wobei es sich bei dem Objekt um ein Symbol (S), insbesondere einen Pfeil handelt, das eine Richtung (R) definiert und die Steuerung (20) das Bild (11) entsprechend dieser Richtung (R) automatisch ausrichtet, und/oder wobei durch die Objekterfassung eine OP-Situation erkannt wird, wobei die Steuerung das Bild entsprechend der erkannten OP-Situation ausrichtet.

11. Operationsleuchtenanordnung (1) nach Anspruch 9, wobei es sich bei dem Objekt um eine Person, insbesondere einen Operateur (O) handelt, wobei die Steuerung (20) das Bild (11) bevorzugt entsprechend einer Blickrichtung (B) der Person und/oder einer Geste der Person automatisch ausrichtet und/oder wobei es sich bei dem Objekt um ein medizinisches Instrument und/oder um eine Anordnung aus mehreren medizinischen Instrumenten handelt, wobei die Steuerung (20) das Bild (11) entsprechend der Ausrichtung des Instruments oder der Anordnung von Instrumenten ausrichtet.

12. Operationsleuchtenanordnung (1) nach einem der vorangegangenen Ansprüche, wobei in mindestens einem Betriebsmodus das Bild (11) manuell ausrichtbar ist und/oder wobei die Steuerung (20) in mindestens einem Betriebsmodus anhand von Signalen einer manuell bedienbaren Eingabevorrichtung (40) das Bild (11) ausrichtet.

13. Operationsleuchtenanordnung (1) nach einem der vorangegangenen Ansprüche, wobei die Operationsleuchtenanordnung (1) mindestens zwei Betriebsmodi umfasst, wobei in einem ersten Betriebsmodus das Ausrichten des Bildes (11) automatisch erfolgt, insbesondere anhand von Signalen eines Neigungssensors (21) und/oder mittels einer Objekterfassung (30), und in einem zweiten Betriebsmodus das Ausrichten des Bildes (11) manuell erfolgt.

14. Set aus einer Operationsleuchtenanordnung (1) nach einem der vorangegangenen Ansprüche und einem Symbol (S), welches von einer Objekterfassung (30) der Steuerung (20) erfasst und zum Ausrichten des Bildes (11) herangezogen wird, wobei das Symbol (S) bevorzugt ein sterilisierbares Element darstellt.

15. Verfahren zum Betrieb einer Operationsleuchtenanordnung (1), insbesondere einer Operationsleuchtenanordnung (1) nach einem der vorangegangenen Ansprüche, wobei die Operationsleuchtenanordnung (1) mindestens eine Operationsleuchte (2) und mindestens eine Kamera (10) umfasst, wobei das Verfahren umfasst:
- Beleuchten eines Operationsfeldes (F) mittels der Operationsleuchte (2);
- Aufnehmen eines Bildes (11) des Operationsfeldes (F) mittels der Kamera (10); und
- automatisches Ausrichten des Bildes (11) der Kamera (10), insbesondere durch automatisches Drehen um eine optische Hauptachse (OH) der Kamera (10).
